# EUROPEAN PATENT SPECIFICATION

(11) **EP 2 799 095 B1**
(45) Date of publication and mention of the grant of the patent: **12.09.2018**
(21) Application number: 12861952.5
(22) Date of filing: 04.10.2012
(51) Int. Cl.: A61L 27/54, A61L 27/28, A61L 27/50, A61C 8/02, A61K 31/67, A61C 8/00, A61K 38/18, A61K 31/663, A61K 31/675

(54) **DENTAL IMPLANT HAVING ENHANCED EARLY STABILITY AND METHOD FOR MANUFACTURING SAME**
ZAHNIMPLANTAT MIT VERBESSERTER FRÜHFESTIGKEIT UND HERSTELLUNGSVERFAHREN DAFÜR
IMPLANT DENTAIRE AYANT UNE STABILITÉ PRÉCOCE AMÉLIORÉE ET SON PROCÉDÉ DE PRODUCTION

(30) Priority: 27.12.2011 KR 20110143049
(43) Date of publication of application: 05.11.2014
(73) Proprietor: Osstemimplant Co., Ltd., Seoul 153-759 (KR)
(72) Inventor: KIM, Su Kyoung, Busan 611-085 (KR); KANG, Eun Jung, Busan 608-020 (KR); SONG, Ju Dong, Busan 609-310 (KR); EOM, Tae Gwan, Busan 612-030 (KR); CHOI, Kyoo Ok, Seoul 153-759 (KR)
(74) Representative: Caspary, Karsten
(86) International application number: PCT/KR2012/008000
(87) International publication number: WO 2013/100329

(56) References cited:
- EP-A1- 2 014 319
- WO-A1-2011/128424
- KR-A- 20080 111 243
- KR-A- 20100 017 882
- KR-A- 20100 057 796
- KR-A- 20110 082 658
- KR-A- 20110 082 658
- KR-B1- 100 736 826

## Description

### [Technical Field]

The present invention relates to a dental implant with enhanced initial stability and a method for manufacturing the same and, more particularly, to a dental implant and a method for manufacturing the same, which can ensure initial stability and fixation of the implant by inhibiting early bone resorption after implant procedure and, at the same time, enhance osseointegration at an implant-bone interface during bone growth by controlling the bone remodeling rate.

### [Background Art]

Dental implants (hereinafter simply referred to as implants) are artificial teeth, which can be used to permanently replace missing teeth, and are widely used to restore the masticatory function of partially or completely edentulous patients. Therefore, the dental implants should be designed to functionally act as actual teeth and, at the same time, to properly distribute the load applied to the teeth, thus enabling long-term use.

The success rate and long-term prognosis of the implant depend on the stability, i.e., fixation that is most affected by bone mass and bone quality of patients. The stability of the implant is expressed by the sum of primary stability (i.e., mechanical stability) that occurs when the implant is brought into contact with the surrounding bone and secondary stability (i.e., biological stability) that results from the formation of new bone tissue and the occurrence of osseointegration after implantation of the implant. Moreover, due to the nature of the process in which the stability of the implant is obtained, an area where the stability of the implant decreases is inevitably formed, and thus the time for which the implant is particularly vulnerable to external loads is present.

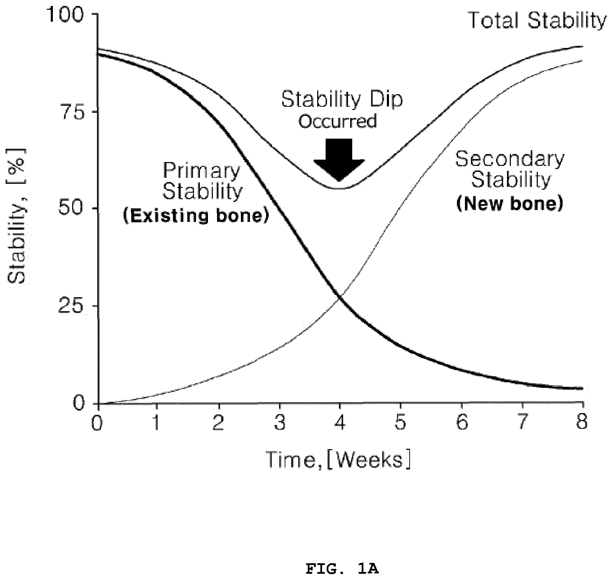

That is, as shown in FIG. 1A, when the implant is implanted in the alveolar bone, bone resorption to form new bone occurs first in the existing bone, which decreases the stability of the implant, and at the same time, new bone is formed around the implant, which in turn increases the stability of the implant due to osseointegration between the implant and the alveolar bone.

The area where the stability of the implant decreases varies depending on the implant design or surface treatment, but is a common phenomenon that occurs in all dental implants, and the initial stability of the implant is most affected at this time.

Therefore, when the initial stability, which is important for successful osseointegration, is difficult to ensure due to low bone mass and poor bone quality of patients, it may cause early failure of the implant. Moreover, when an excessive load is applied to the implant with low initial stability, the osseointegration may be delayed due to minute vibrations, and thus delayed implantation, in which the load is applied after 3 to 6 months for bone growth, is used in conventional dental implants. That is, according to the conventional implant surface treatment technology, early loading of the implant increases the failure rate of the implant, and when the delayed implantation is performed to prevent this, the implant procedure is extended, which is very problematic.

In order to solve the above problems and enhance the mechanical stability between the implant and the bone, various attempts have been made to ensure the initial stability of the implant by changing the length or diameter of the implant or by introducing a special design for facilitating self-tapping. Moreover, in order to enhance the biological stability between the implant and the bone by osteogenesis, various methods have been attempted to enhance the initial stability of the implant by applying bone growth factors, extracellular matrix molecules, polymer carriers, etc. to the implant.

As such, various methods have been attempted to ensure the initial stability of the implant, but a plan to ensure the initial stability after implantation of the implant has not been developed so far, which makes it difficult to enable early loading and reduce treatment period.

WO 2011/128424 A1 discloses methods, uses and articles in the field of orthopedic and dental implants, particularly compositions and methods for improving the osseointegration of such implants. The dental implants comprise a coating having an osteoclast activity inhibitor and a bone anabolic agent.

KR 2011 0082658 A describes a titanium-based alloy implant, whose surface is subjected to an electrochemical treatment. The surface is oxidized and titanium dioxide nanotubes are formed. An osteoclast activity inhibitor coating is deposited onto the roughened surface. The inhibitor coating comprises bisphosphonate and an antibiotic.

EP 2 014 319 A1 discloses a dental implant having a roughened surface and comprising a coating which includes strontium ions.

### [Disclosure]

### [Technical Problem]

Accordingly, the present invention has been made to solve the above-described problems, and an object of the present invention is to provide a dental implant and a method for manufacturing the same, which can ensure initial stability after implantation of the implant and enhance osseointegration at an implant-bone interface during bone growth, thus enabling early loading and reducing treatment period.

### [Technical Solution]

To achieve the above objects, the present invention provides a dental implant comprising the features of claim 1.

Moreover, the present invention provides a method for manufacturing a dental implant, the method comprising the features of claim 3.

Meanwhile, the osteoclast activity inhibitor for application to alveolar bone is applied to the surface of the alveolar bone, which is in contact with a dental implant, before implantation of the implant to inhibit bone resorption of the alveolar bone, thus enhancing initial stability of the implant and osseointegration at an implant-bone interface.

The osteoclast activity inhibitor is used to inhibit early bone resorption of the alveolar bone and comprises a therapeutic agent for osteoporosis, and the therapeutic agent for osteoporosis comprises at least one selected from the group consisting of bisphosphonate, transforming growth factor β1 (TGFβ1), calcitonin, a selective estrogen receptor modulator (SERM), osteoprotegerin (OPG), a receptor activator of nuclear factor-κB ligand (RANKL) inhibitor, disintegrin, a cysteine-protease inhibitor, an H⁺-ATPase inhibitor, and strontium salt.

Furthermore, the osteoclast activity inhibitor coating film further comprises a bone growth factor such as BMP-2, PEP7, etc. to promote osseointegration of the implant.

Moreover, the bisphosphonate used as the osteoclast activity inhibitor collectively includes first-, second- and third-generation bisphosphonates with a P-C-P backbone and may comprise at least one selected from the group consisting of etidronate, clodronate, tiludronate, pamidronate, alendronate, risedronate, ibandronate, zolendronate, and pharmaceutically acceptable salts, esters, and acids thereof.

In addition, the hydrophilization treatment may be performed by plasma or ultraviolet treatment on the surface of the dental implant.

### [Advantageous Effects]

According to the present invention, it is possible to ensure initial stability and fixation of the implant after implantation by coating the surface of the dental implant with an osteoclast activity inhibitor that inhibits the activity of osteoclasts and, at the same time, enhance osseointegration at an implant-bone interface during bone growth by controlling the bone remodeling rate, thus enabling early loading after implant procedure and reducing treatment period.

### [Description of Drawings]

FIG. 1A is a diagram showing the principle of decreased initial stability after implant procedure.
FIG. 1B is a diagram showing the principle of increased initial stability after implant procedure according to the present invention.
FIG. 2 is a flowchart showing the preparation and implantation of an implant in accordance with an embodiment of the present invention.
FIGS. 3 and 4 show the results of evaluation of initial stability for 6 weeks after implantation of implants in the mandible and tibia of micropigs.
FIG. 5 shows the measurement results of removal torque after 16 days for bone growth after implantation of implantsin the tibia of micropigs.
FIG. 6 shows the measurement results of removal torque after 16 days for bone growth after implantation of implants in accordance with an embodiment of the present invention in the tibia of micropigs.

### [Mode for Invention]

The dental implant and the method for manufacturing the same in accordance with aspects of the present invention will be described in detail with reference to the accompanying drawings.

First, as used herein, the term "implant" refers to a substitute for restoring lost body tissue, and the term "dental implant" refers to a substitute intended to restore the original function of a tooth in a manner that a fixture is embedded and integrated in the alveolar bone, from which a natural dental root is removed, to replace the root of a missing tooth and then an artificial tooth is fixed onto the top of the fixture.

In particular, in the present invention, the surface of the dental implant refers to the surface of the fixture that can be integrated in the alveolar bone and may be made of titanium or a titanium alloy comprising titanium and at least one of aluminum, tantalum, niobium, vanadium, zirconium, platinum, magnesium, and sodium.

As shown in FIG. 1A, when the implant is implanted in the alveolar bone, bone resorption to form new bone occurs first in the existing bone, which decreases the stability of the implant, and at the same time, new bone is formed around the implant, which in turn increases the stability of the implant due to osseointegration between the implant and the alveolar bone. Due to the nature of the process in which the stability of the implant is obtained, an area where the stability of the entire implant decreases is inevitably formed, and thus the time for which the implant is particularly vulnerable to external loads is present. Therefore, when an excessive load is applied to the implant with low initial stability, the osseointegration may be delayed due to minute vibrations.

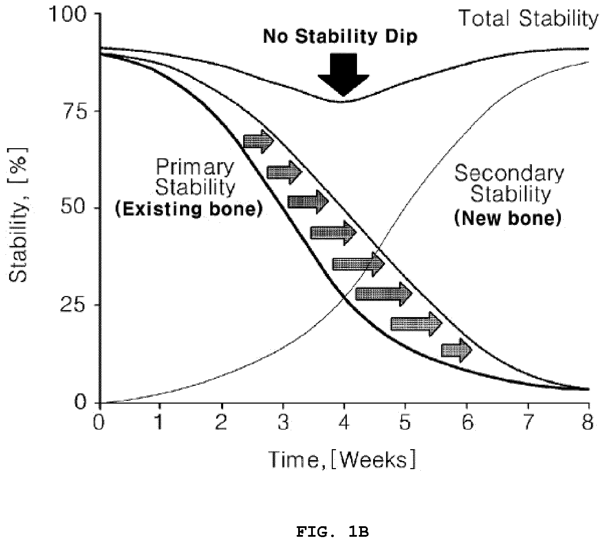

In the present invention, in order to solve these problems, the osteoclast activity inhibitor coating film, which can inhibit early bone resorption of the alveolar bone, is formed on the implant surface. When the implant is coated with the osteoclast activity inhibitor and then implanted in the alveolar bone, the osteoclast activity inhibitor coated on the implant surface is released from the alveolar bone to the surrounding bone. The released osteoclast activity inhibitor is adsorbed onto the surrounding bone to inhibit the activity of osteoclasts, which delays bone remodeling at an implant-bone interface fixed to the alveolar bone to alleviate the decrease in primary stability of the implant, thus increasing the initial stability of the implant as shown in FIG. 1B. Moreover, the implant coated with the osteoclast activity inhibitor of the present invention maintains the increase in the initial stability due to an increased osseointegration period, thus enhancing the osseointegration at the implant-bone interface.

That is, the implantation of the implant coated with the osteoclast activity inhibitor according to the present invention can ensure the initial stability and fixation of the implant by minimizing the area where the stability of the implant decreases and, at the same time, enable early loading of the implant, thus preventing delay in osseointegration due to minute vibrations and reducing the osseointegration period.

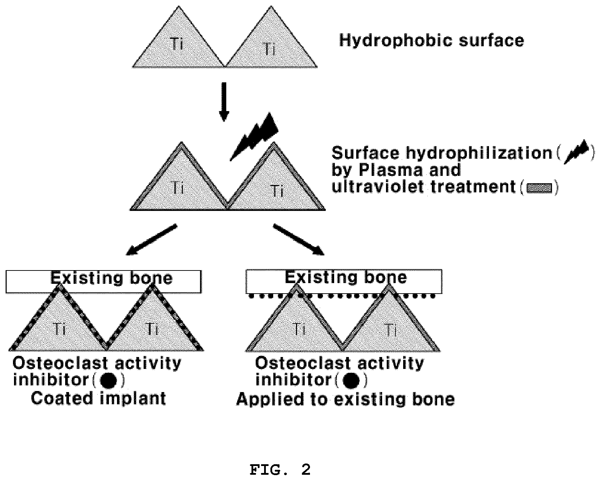

Moreover, as shown in FIG. 2, the osteoclast activity inhibitor may not be coated directly on the implant, but may be applied directly to the surface of the alveolar bone, which is in contact with the implant, before implantation of the implant, thus obtaining the same effect. Moreover, the osteoclast activity inhibitor may be applied in the form of a solution or in a dried state to the implant surface or the alveolar bone surface to facilitate its release and absorption.

The osteoclast activity inhibitor comprises a variety of materials, which inhibit early bone resorption of the alveolar bone, namely a therapeutic agent for osteoporosis. The therapeutic agent for osteoporosis comprises at least one selected from the group consisting of bisphosphonate, transforming growth factor β1 (TGFβ1), calcitonin, a selective estrogen receptor modulator (SERM), osteoprotegerin (OPG), a receptor activator of nuclear factor-κB ligand (RANKL) inhibitor, disintegrin, a cysteine-protease inhibitor, an H⁺-ATPase inhibitor, and strontium salt.

Moreover, the bisphosphonate used as the osteoclast activity inhibitor collectively includes first-, second- and third-generation bisphosphonates with a P-C-P backbone and may comprise at least one selected from the group consisting of etidronate, clodronate, tiludronate, pamidronate, alendronate, risedronate, ibandronate, zolendronate, and pharmaceutically acceptable salts, esters, and acids thereof.

Furthermore, the osteoclast activity inhibitor coating film further comprises a bone growth factor such as BMP-2, PEP7, etc. to promote osseointegration of the implant.

Meanwhile, before the step of coating the osteoclast activity inhibitor on the implant surface, the implant surface is further subjected to the step of roughening the surface and the step of hydrophilization treatment so as to further enhance the osseointegration. The roughening may be performed by various methods such as blasting, resorbable blasting media, acid etching, alkali etching, titanium plasma spray, sandblasting with large grit and acid treatment, anodizing, laser surface processing, etc., and the roughened implant surface has an increased surface area, which enhances the osseointegration of the implant.

Moreover, the hydrophilization treatment of the implant surface may be performed by various methods that can remove organic contaminants from the surface, and as an example, plasma treatment such as RFGD, O₂, and room temperature plasma or ultraviolet treatment may be used.

Next, the effect of the present invention will be described in detail with reference to the following Examples. However, the following Examples are merely illustrative of one or more detailed examples, and the scope of the present invention is not limited to the following Examples.

### Example 1: Preparation of dental implant subjected to hydrophilization treatment and coated with osteoclast activity inhibitor

Machined titanium implants were blasted with Al₂O₃ powder with a particle size of 1 mm or less at a blast pressure of 1 to 10 atm for 1 to 60 seconds. Macro- & micro-morphology was given to the implant surface by acid treatment using a mixed acid solution, and then the acid-etched dental titanium implant was washed with ethanol for 30 minutes and with distilled water by ultrasonication for 30 minutes and then dried.

In order to impart hydrophilicity to the implants which were subjected to the above processes, the titanium surface was hydrophilized by plasma treatment (RFGD, O₂, etc.) for 1 minutes and light radiation (ultraviolet rays, ultraviolet-ozone, etc.) for 5 minutes. Then, a 10 ml solution of 40 mg Alendronate, 40 mg Zolendronate, 1 mg BMP-2, an 40 mg Alendronate+1mg BMP-2 was uniformly applied to the surface, and the prepared implants, in which the solution was not dried, were used in the following Examples 2, 3 and 4.

### Example 2 (not according to the invention): Animal experiments for measurement of implant stability quotients to evaluate initial stability of dental implants coated with osteoclast activity inhibitor

In order to determine implant stability quotients (ISQs), the dental implants coated with alendronate prepared in Example 1 were implanted in the mandible and tibia of micropigs, and then the resonance frequency analysis (RFA) values were measured for ISQs using Osstell™ Mentor (Integration Diagnostics Ltd., Goteborg, Sweden) and Smartpeg™ (Integration Diagnostics Ltd., Goteborg, Sweden) at 0, 0.5, 1, 1.5, 2, 4, and 6 weeks, respectively. At this time, implants that were not coated with the osteoclast activity inhibitor were used as the negative control group, and implants that were subjected to pre-treatment for hydrophilizing the titanium surface and coated with alendronate were used as the experimental group.

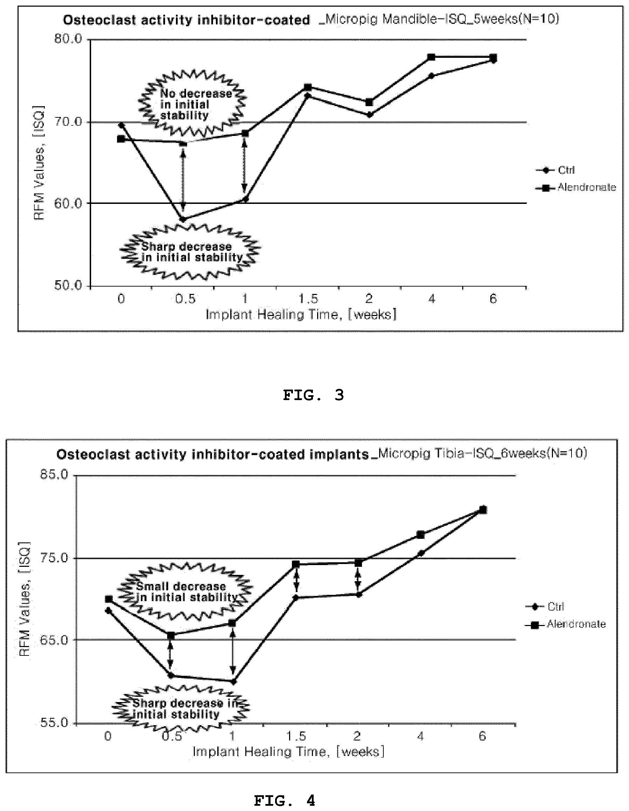

As can be seen from FIGS. 3 and 4, there was little decrease in ISQ values or the degree of the decrease was very lower in the experimental group than in the negative control group, from which it was confirmed that the initial stability of the implant increased.

### Example 3 (not according to the invention): Animal experiments for measurement of osseointegration at implant-bone interface to evaluate initial stability of dental implants coated with osteoclast activity inhibitor

In order to determine the osseointegration at the implant-bone interface, the dental implants coated with alendronate and zolendronate prepared in Example 1 were implanted in the tibia of micropigs, and then the removal torques were measured after 16 days for bone growth. At this time, implants that were not coated with the osteoclast activity inhibitor were used as the negative control group, and implants that were subjected to pre-treatment for hydrophilizing the titanium surface and coated with alendronate and zolendronate were used as the experimental group.

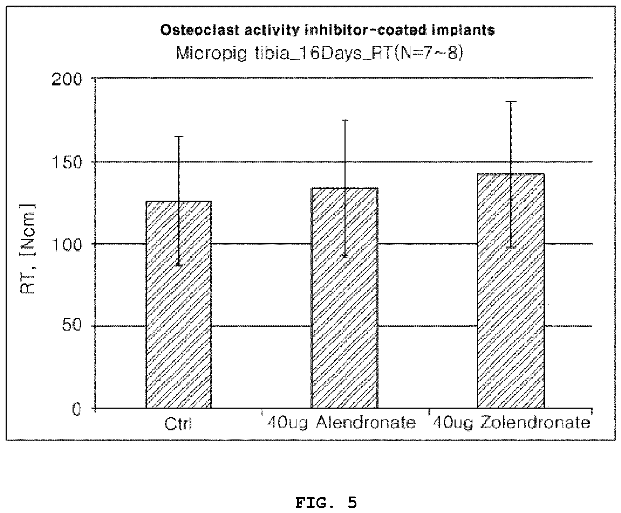

As shown in FIG. 5, the removal torque was increased by about 6 to 13% in the experimental group compared to the negative control group, from which it was confirmed that the osseointegration at the implant-bone interface increased in the implants coated with the osteoclast activity inhibitor.

### Example 4: Animal experiments for measurement of osseointegration at implant-bone interface to evaluate initial stability of dental implants coated with osteogenic protein and osteoclast activity inhibitor

In order to determine the osseointegration at the implant-bone interface, the dental implants coated with rhBMP-2 and alendronate prepared in Example 1 were implanted in the tibia of micropigs, and then the removal torques were measured after 16 days for bone growth. At this time, implants that were coated only with rhBMP-2, an osteogenic protein, were used as the negative control group, and implants that were subjected to pre-treatment for hydrophilizing the titanium surface and coated with rhBMP-2 and alendronate were used as the experimental group.

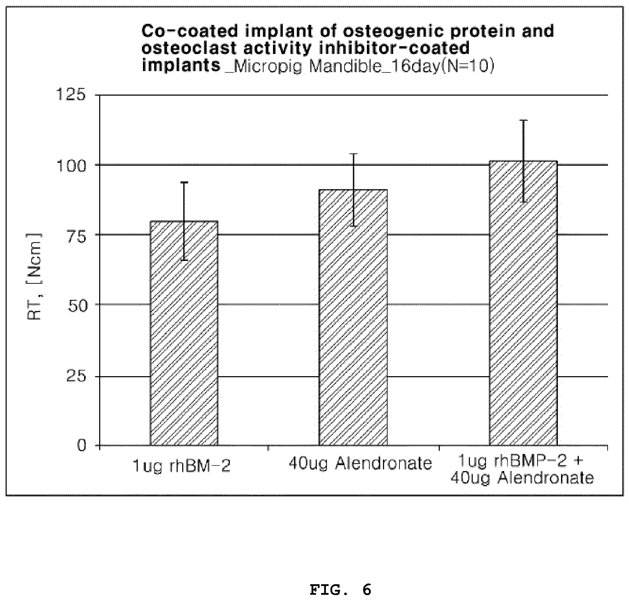

As shown in FIG. 6, the removal torque was increased by about 23% in the experimental group compared to the negative control group, from which it was confirmed that the osseointegration at the implant-bone interface was increased by the use of the osteoclast activity inhibitor in combination with the osteogenic protein.

## Claims

1. A dental implant comprising:
a roughened surface; and
an osteoclast activity inhibitor coating film which is formed on the surface of the dental implant to enhance initial stability of the implant and osseointegration at an implant-bone interface,
wherein during implantation of the implant, the osteoclast activity inhibitor is released from the coating film to surrounding alveolar bone, the related osteoclast activity inhibitor inhibiting bone resorption of the surround alveolar bone to control early bone remodeling at the implant-bone interface, thus enhancing initial stability of the implant,
wherein the osteoclast activity inhibitor comprises a therapeutic agent for osteoporosis,
wherein the therapeutic agent for osteoporosis comprises at least one selected from the group consisting of bisphosphonate, transforming growth factor β1 (TGFβ1), calcitonin, a selective estrogen receptor modulator (SERM), osteoprotegerin (OPG), a receptor activator of nuclear factor-κB ligand (RANKL) inhibitor, disintegrin, a cysteine-protease inhibitor, an H+-ATPase inhibitor, and strontium salt, and
wherein the osteoclast activity inhibitor coating film further comprises BMP-2 or PEP-7 which is a bone growth factor.

2. The dental implant of claim 1, wherein the bisphosphonate comprises at least one selected from the group consisting of etidronate, clodronate, tiludronate, pamidronate, alendronate, risedronate, ibandronate, zolendronate, and pharmaceutically acceptable salts, esters, and acids thereof.

3. A method for manufacturing a dental implant, the method comprising the steps of:
roughening a surface of a dental implant;
subjecting the roughened surface of the dental implant to hydrophilization treatment; and
forming an osteoclast activity inhibitor coating film on the surface of the hydrophilized surface of the dental implant,
wherein during implantation of the implant, the osteoclast activity inhibitor is released from the coating film to surrounding alveolar bone, the related osteoclast activity inhibitor inhibiting bone resorption of the surround alveolar bone to control early bone remodeling at the implant-bone interface, thus enhancing initial stability of the implant,
wherein the osteoclast activity inhibitor comprises a therapeutic agent for osteoporosis,
wherein the therapeutic agent for osteoporosis comprises at least one selected from the group consisting of bisphosphonate, transforming growth factor β1 (TGFβ1), calcitonin, a selective estrogen receptor modulator (SERM), osteoprotegerin (OPG), a receptor activator of nuclear factor-κB ligand (RANKL) inhibitor, disintegrin, a cysteine-protease inhibitor, an H+-ATPase inhibitor, and strontium salt,
wherein the osteoclast activity inhibitor coating film further comprises BMP-2 or PEP-7 which is a bone growth factor, and
wherein the hydrophilization treatment is performed by plasma or ultraviolet treatment on the surface of the dental implant.

4. The method of claim 3, wherein the bisphosphonate comprises at least one selected from the group consisting of etidronate, clodronate, tiludronate, pamidronate, alendronate, risedronate, ibandronate, zolendronate, and pharmaceutically acceptable salts, esters, and acids thereof.

## Patentansprüche

1. Zahnimplantat mit:
einer gerauten Oberfläche; und
einer Osteoklastenaktivitätsinhibitor-Beschichtung, die an der Oberfläche des Zahnimplantats ausgebildet ist, um die Anfangsfestigkeit des Implantats und die Osseointegration an einer Implantat-Kochen-Schnittstelle zu verbessern,
wobei während der Implantation des Implantats der Osteoklastenaktivitätsinhibitor von der Beschichtung auf den umliegenden Alveolarknochen abgegeben wird, wobei der betreffende Osteoklastenaktivitätsinhibitor die Knochenresorption des umliegenden Alveolarknochens hemmt, um eine frühzeitige Knochenveränderung an der Implantat-Knochen-Schnittstelle zu steuern, so dass die Frühfestigkeit des Implantats verbessert wird,
wobei der Osteoklastenaktivitätsinhibitor einen therapeutischen Wirkstoff für Osteoporose aufweist,
wobei der therapeutische Wirkstoff für Osteoporose mindestens einen Stoff aufweist, der aus der Gruppe ausgewählt ist, die aus Bisphosphonat, einem transformierenden Wachstumsfaktor β1 (TGFβ1), Calcitonin, einem selektiven Östrogenrezeptormodulator (SERM), Osteoprotegerin (OPG), einem Receptor-Activator-of-Nuclear-Factor-kB-Ligand (RANKL) - Inhibitor, Disintegrin, einem Cysteinproteaseinhibitor, einem H+-ATPase-Inhibitor, und Strontiumsalz besteht, und
wobei die Osteoklastenaktivitätsinhibitor-Beschichtung weiterhin BMP-2 oder PEP-7 aufweist, das ein Knochenwachstumsfaktor ist.

2. Zahnimplantat nach Anspruch 1, wobei das Bisphosphonat mindestens einen Stoff aufweist, der aus der Gruppe ausgewählt ist, die aus Etidronat, Clodronat, Tiludronat, Pamidronat, Alendronat, Risedronat, Ibandronat, Zolendronat, und pharmazeutisch verträglichen Salzen, Ester, und Säuren davon besteht.

3. Verfahren zur Herstellung eines Zahnimplantats, wobei das Verfahren die folgenden Schritte aufweist:
Aufrauen einer Oberfläche eines Zahnimplantats;
Unterziehen der gerauten Oberfläche des Zahnimplantats einer Hydrophilisierungsbehandlung; und
Bilden einer Osteoklastenaktivitätsinhibitor-Beschichtung an der Oberfläche der hydrophilisierten Oberfläche des Zahnimplantats,
wobei während der Implantation des Implantats der Osteoklastenaktivitätsinhibitor von der Beschichtung auf den umliegenden Alveolarknochen abgegeben wird, wobei der betreffende Osteoklastenaktivitätsinhibitor die Knochenresorption des umliegenden Alveolarknochens hemmt, um einen frühzeitigen Knochenumbau an der Implantat-Knochen-Schnittstelle zu regulieren, sodass die Frühfestigkeit des Implantats verbessert wird,
wobei der Osteoklastenaktivitätsinhibitor einen therapeutischen Wirkstoff für Osteoporose aufweist,
wobei der therapeutische Wirkstoff für Osteoporose mindestens einen Stoff aufweist, der aus der Gruppe ausgewählt ist, die aus Bisphosphonat, einem transformierenden Wachstumsfaktor β1 (TGFβ1), Calcitonin, einem selektiven Östrogenrezeptormodulator (SERM), Osteoprotegerin (OPG), einem Receptor-Activator-of-Nuclear-Factor-kB-Ligand (RANKL) - Inhibitor, Disintegrin, einem Cysteinproteaseinhibitor, einem H+-ATPase-Inhibitor, und Strontiumsalz besteht, und
wobei die Osteoklastenaktivitätsinhibitor-Beschichtung weiterhin BMP-2 oder PEP-7 aufweist, welches ein Knochenwachstumsfaktor ist.

4. Verfahren nach Anspruch 3, wobei das Bisphosphonat mindestens einen Stoff aufweist, der aus der Gruppe ausgewählt ist, die aus Etidronat, Clodronat, Tiludronat, Pamidronat, Alendronat, Risedronat, Ibandronat, Zolendronat, und pharmazeutisch verträglichen Salzen, Ester, und Säuren davon besteht.

## Revendications

1. Implant dentaire comprenant :
une surface rugueuse ; et
un film de revêtement inhibiteur de l'activité ostéoclastique qui est formé sur la surface de l'implant dentaire pour améliorer la stabilité initiale de l'implant et l'ostéo-intégration à une interface implant-os,
dans lequel pendant l'implantation de l'implant, l'inhibiteur de l'activité ostéoclastique est libéré par le film de revêtement dans l'os alvéolaire environnant, l'inhibiteur de l'activité ostéoclastique associé inhibant la résorption osseuse de l'os alvéolaire environnant pour contrôler le remodelage osseux précoce à l'interface implant-os, améliorant, de ce fait, la stabilité initiale de l'implant,
dans lequel l'inhibiteur de l'activité ostéoclastique comprend un agent thérapeutique pour l'ostéoporose,
dans lequel l'agent thérapeutique pour l'ostéoporose comprend au moins un(e) choisi (e) dans le groupe consistant en bisphosphonate, facteur de croissance transformant β1 (TGFβ1), calcitonine, modulateur sélectif des récepteurs aux oestrogènes (MSRO), ostéoprotégérine (OPG), inhibiteur du ligand du récepteur activateur du facteur kappa b nucléaire (RANKL), désintégrine, inhibiteur d'une cystéine-protéase, inhibiteur de H+-ATPase, et sel de strontium, et
dans lequel le film de revêtement inhibiteur de l'activité ostéoclastique comprend, en outre, BMP-2 ou PEP-7, qui est un facteur de croissance osseuse.

2. Implant dentaire de la revendication 1, dans lequel le bisphosphonate comprend au moins un choisi dans le groupe consistant en étidronate, clodronate, tiludronate, pamidronate, alendronate, risédronate, ibandrtonate, zolendronate, et des sels acceptables sur le plan pharmaceutique, des esters, et des acides de ceux-ci.

3. Procédé de fabrication d'un implant dentaire, le procédé comprenant les étapes de :
dégrossissage d'une surface d'un implant dentaire ;
soumission de la surface rugueuse de l'implant dentaire à un traitement d'hydrophilisation ; et
formation d'un film de revêtement inhibiteur de l'activité ostéoclastique sur la surface hydrophilisée de l'implant dentaire,
dans lequel, pendant l'implantation de l'implant, l'inhibiteur de l'activité ostéoclastique est libérée par le film de revêtement dans l'os alvéolaire environnant, l'inhibiteur de l'activité ostéoclastique associé inhibant la résorption osseuse de l'os alvéolaire environnant pour contrôler le remodelage osseux précoce à l'interface implant-os, améliorant, de ce fait, la stabilité initiale de l'implant,
dans lequel l'inhibiteur de l'activité ostéoclastique comprend un agent thérapeutique pour l'ostéoporose,
dans lequel l'agent thérapeutique pour l'ostéoporose comprend au moins un(e) choisi (e) dans le groupe consistant en bisphosphonate, facteur de croissance transformant β1 (TGFβ1), calcitonine, modulateur sélectif des récepteurs aux oestrogènes (MSRO), ostéoprotégérine (OPG), inhibiteur du ligand du récepteur activateur du facteur kappa b nucléaire (RANKL), désintégrine, inhibiteur d'une cystéine-protéase, inhibiteur de H+-ATPase, et sel de strontium, et
dans lequel le film de revêtement inhibiteur de l'activité ostéoclastique comprend, en outre, BMP-2 ou PEP-7, qui est un facteur de croissance osseuse
dans lequel le traitement d'hydrophilisation est réalisé par traitement par plasma ou ultraviolet sur la surface hydrophilisée de l'implant dentaire.

4. Procédé de la revendication 3, dans lequel le bisphosphonate comprend au moins un choisi dans le groupe consistant en étidronate, clodronate, tiludronate, pamidronate, alendronate, risédronate, ibandrtonate, zolendronate, et des sels acceptables sur le plan pharmaceutique, des esters, et des acides de ceux-ci.
